# EUROPEAN PATENT SPECIFICATION

(11) **EP 1 430 896 B1**
(45) Date of publication and mention of the grant of the patent: **11.11.2009**
(21) Application number: 02767998.4
(22) Date of filing: 20.09.2002
(51) Int. Cl.: A61K 31/465, A61K 9/70, A61K 47/10, A61K 47/32, A61K 47/34, A61K 47/36, A61K 47/38, A61K 47/46, A61P 25/34, A61K 47/26

(54) **NICOTINE-CONTAINING FILM PREPARATION**
NIKOTIN ENTHALTENDE FILMZUBEREITUNG
PREPARATION POUR FILM CONTENANT DE LA NICOTINE

(30) Priority: 25.09.2001 JP 2001292616
(43) Date of publication of application: 23.06.2004
(73) Proprietor: KYUKYU PHARMACEUTICAL CO., LTD., Chuo-ku, Tokyo 103-0023 (JP)
(72) Inventor: AWAMURA, T., c/o Kyukyu Pharmaceutical Co., Ltd., Imizu-gun, Toyama 939-0351 (JP); SAWAI, Y., c/o Kyukyu Pharmaceutical Co., Ltd., Imizu-gun, Toyama 939-0351 (JP)
(74) Representative: Helbing, Jörg
(86) International application number: PCT/JP2002/009682
(87) International publication number: WO 2003/026654

(56) References cited:
- EP-A1- 0 781 546
- WO-A-00/18365
- WO-A-96/00072
- WO-A1-95/24172
- WO-A1-99/04764
- JP-A- 3 209 326
- JP-A- 3 209 327
- JP-A- 4 173 730
- JP-A- 63 310 818

## Description

### Technical Field

The present invention relates to a nicotine-containing film preparation for application to the oral mucous membrane, which is used as an aid for smoking cessation or moderation in smoking, and more particularly to a film preparation having a three-layered structure comprising a nicotine-containing drug layer and coating layers provided on both faces thereof, the coating layers being able to adhere to the oral mucous membrane.

### Background Art

As for preparations relating to nicotine administration through the oral mucous membrane, certain preparations have been known. For example, there have been known tablets for intraoral or sublingual administration (for example, refer to patent documents 1 and 2), lozenges (for example, refer to patent documents 3 and 4), hydrocarbon powder matrixes (for example, refer to patent document 5), troches (for example, refer to patent document 6: specifically tablet type troches), capsules (for example, refer to patent document 7), saliva-soluble preparations including patches in claims, but no specific patch examples are seen at all (for example, refer to patent documents 8 and 9) and patches for the oral mucous membrane (for example, refer to patent documents 10, 11, 12 and 13).

### Prior Art

patent Document 1
   International Patent Unexamined Publication No. 6-502622 (pages 1 and 2)
Patent Document 2
   International Patent Unexamined Publication No. 5-504949 (pages 1 and 2)
Patent Document 3
   International Patent Unexamined Publication No. 9-505553 (pages 1 and 2)
Patent Document 4
   Japanese Patent Unexamined Publication No. 9-286729 (pages 1 and 2)
Patent Document 5
   Japanese Patent Unexamined Publication No. 9-286729 (pages 1 and 2)
Patent Document 6
   International Patent Unexamined Publication No. 2000-504028 (pages 1 and 2)
Patent Document 7
   Japanese Patent No. 2602043 (pages 1 and 2)
Patent Document 8
   Japanese Patent No. 2958663 (pages 1 and 2)
Patent Document 9
   Japanese Patent Unexamined Publication No. 11-315023 (pages 1 and 2)
Patent Document 10
   Japanese Patent Unexamined Publication No. 3-209626 (pages 1 and 2)
Patent Document 11
   Japanese Patent Unexamined Publication No. 3-209627 (pages 1 and 2)
Patent Document 12
   Japanese Patent Unexamined Publication No. 62-178513 (pages 1 and 2)
Patent Document 13
   Japanese Patent Unexamined Publication No. 63-227522 (pages 1 and 2)

WO 96/00072 discloses a single layer or multi-layer transdermal or transmucosal system containing nicotine and caffeine. The multi-layer system may comprise an adhesive layer, a reservoir layer and a backing component.

WO 00/18365 discloses edible films with mainly composed of pullulan as film-forming polymer and containing pharmaceutical agents like nicotine.

Although the conventional nicotine administration preparations through the oral mucous membrane have each been cogitated, many have a feeling that something is wrong. Further, the preparations are easily crunched or swallowed, or complicated in their structure. Accordingly, there is the problem of complicated manufacturing methods. Furthermore, the preparations have insufficient sustained release, poor reproducibility or the difficulty of controlling sustained release.

When nicotine is allowed to be absorbed through the oral mucous membrane, the dissolution time of a film preparation is not particularly specified. However, when it dissolves too early, the active ingredient is difficult to stay in the oral cavity, resulting in insufficient absorption. Admitting that the feeling that something is wrong is decreased, adhesion to the upper jaw for a period longer than necessary raises the problem that a disturbance is unfavorably caused by the taste of nicotine itself etc. in the case of drinking and eating during that.

The present invention provides a nicotine film preparation easily adhesive to the mucous membrane, having a suitable sustained release of nicotine and good in reproducibility thereof, rich in flexibility and having elasticity, and having no feeling that something is wrong in application.

### Disclosure of the Invention

In order to solve the above-mentioned problems, that is to say, in order to provide a nicotine-containing film preparation having suitable sustained release and no feeling that something is wrong in application, the present inventors have made intensive studies. As a result, the inventors have discovered that a film preparation having suitable sustained release, having no feeling that something is wrong in application, and easily adhesive to the mucous membrane is obtained by combining coating layers having a specified peel strength and solubility and a nicotine-containing layer having a specified peel strength and solubility different from those of the coating layers so as to give a definite thickness, thus completing the present invention.

That is to say, the present invention is directed to (1) a film preparation of a three-layer structure consisted of a nicotine-containing film layer and two coating layer films laminated respectively on both sides of the nicotine-containing layer film as a patch for the oral cavity, wherein
(1) the nicotine-containing film layer comprises nicotine and an edible water-soluble polymer and has a 180-degree peel strength in application to a Bakelite plate of 1 g to 30 g/20 mm and a time required for dissolution In water of 3 to 10 min as measured by dissolution of a 1.5 x 1.5 cm test piece of a 100 µm film in 900 mi of purified water at 50 revolutions with a sinker, and
(2) each of the coating layers comprises
   (i)
      (a) at least one of pullulan, a pullulan ether and a pullulan ester as a water-soluble and non-water-absorbing polysaccharide; or
      (b) at least one of pullulan, a pullulan ether and a pullulan ester as a water-soluble and non-water-absorbing polysaccharide and an edible polymer; and
   (ii) a softener, and
each of the coating layers has a 180-degree peel strength in application to a Bakelite plate of 100 g to 500g/20 mm and a time required for dissolution In water of 0.5 to 3 min as measured by dissolution of a 1.5 x 1.5 cm test piece of a 100 µm film in 900 ml of purified water at SO revolutions with a sinker.

(2) the film preparation described in (1), in which the pH of a 0.1% aqueous solution of the preparation is from 3 to 9; (3) the film preparation described in (1), in which the nicotine content in one preparation unit is from 0.1 mg to 5 mg; (4) the film preparation described in (1), in which the time required for dissolution of one preparation unit is from 10 minutes to 30 minutes; (5) the film preparation described in (1), which has a film strength of 2 kg to 5 kg/20 mm; (6) the film preparation described in (1), in which the nicotine-containing layer has a thickness of 250 µm to 500 µm, the coating layer has a thickness of 5 µm to 30 µm, and a total of the three layers have a thickness of 260 µm to 560 µm; (7) the film preparation described in (1), in which the coating layer contains a sucrose fatty acid ester; (8) the film preparation described in (1), in which the nicotine-containing layer contains polyethylene glycol; and (9) the film preparation described in (1), in which the water-soluble and non-water-absorbing polysaccharide is at least one of pullulan, a pullulan ether (methyl, ethyl or propyl) and an pullulan ester (acetate or butyrate), the softener is at least one of glycerol, ethylene glycol, propylene glycol, D-sorbitol, maltitol, mannitol and xylitol, the edible water-soluble polymer is at least one of hydroxypropylcellulose (HPC), hydroxypropylmethyl cellulose (HPMC), hydroxypropylethylcellulose (HPEC), methyl cellulose (MC), water-soluble ethyl cellulose (EC), water-soluble hydroxyethyl cellulose (HEC), carboxymethyl cellulose·sodium (CMC·Na), polyvinyl alcohol (PVA), polyvinyl pyrrolidone (PVP), a carboxyvinyl polymer, sodium alginate and sodium acrylate, and the edible polymer is at least one of hydroxypropylcellulose (HPC), hydroxypropylmethyl cellulose (HPMC), hydroxypropylethyl cellulose (HPEC), methyl cellulose (MC), ethyl cellulose (EC), hydroxyethyl cellulose (HEC), carboxymethyl cellulose·sodium (CMC·Na), polyvinyl alcohol (PVA), polyvinyl pyrrolidone (PVP), a carboxyvinyl polymer, sodium alginate, sodium acrylate, shellac, cellulose acetate phthalate (CAP), polyvinyl acetate, Eudragit^{™}, hydroxypropylmethyl cellulose acetate succinate (HPMCAS) and hydroxypropylmethyl cellulose phthalate (HPMCP).

Previously, Japanese Patent Unexamined Publication No. 3-209626 (patent document 10), Japanese Patent Unexamined Publication No. 3-209627 (patent document 11), Japanese Patent Unexamined Publication No. 62-178513 (patent document 12) and Japanese Patent Unexamined Publication No. 63-227522 (patent document 13) have been mentioned as ones relating to the nicotine-containing patches for the oral mucous membrane.

In all of the preparations of these inventions, water-soluble or water-swellable polymers are used, and there may be particularly no problem in respect to adhesion to the mucous membrane (in the experiments, tests are carried out by sticking the preparations on beaker walls, and this is different from actual use). However, the time for which the preparations have been applied is considered to be too long, and the initial release of drugs is considered to be insufficient. The film preparation of the present invention completely dissolves in 10 to 30 minutes, gives neither a feeling that something is wrong nor an uncomfortable feeling, and allows nicotine to be sufficiently absorbed.

### Best Mode for Carrying Out the Invention

The present invention will be described in more detail below.

In the present invention, a coating layer comprises a water-soluble and non-water-absorbing polysaccharide and/or an edible polymer and a softener. The coating layer is mainly composed of pullulan, the water-soluble and non-water-absorbing polysaccharide, so that it plays a role for protecting a surface of a product. The film preparation of the present invention has thin layers mainly composed of pullulan on both upper and lower faces. Accordingly, when pressed onto the upper jaw, the coating layer dissolves immediately (0.5 to 3 minutes) by moisture of the upper jaw portion, and a nicotine-containing layer comes into contact with the upper jaw and adheres thereto. It does not therefore happen to separate immediately. Then, this nicotine-containing layer dissolves in 10 to 30 minutes to release nicotine, which is absorbed through the mucous membrane.

Although the water-soluble and non-water-absorbing polysaccharides used in the present invention include pullulan, a pullulan ether (methyl, ethyl or propyl) and a pullulan ester (acetate or butyrate), pullulan is suitably used. Although the softeners include glycerol, ethylene glycol, propylene glycol, D-sorbitol, maltitol, mannitol, xylitol, etc., D-sorbitol is suitably used. There is no particular limitation on the edible water-soluble polymer, and examples thereof include hydroxypropylcellulose (HPC), hydroxypropylmethyl cellulose (HPMC), hydroxypropylethylcellulose (HPEC), methyl cellulose (MC), water-soluble ethyl cellulose (EC), water-soluble hydroxyethyl cellulose (HEC), carboxymethyl cellulose·sodium (CMC·Na), polyvinyl alcohol (PVA), polyvinyl pyrrolidone (PVP), a carboxyvinyl polymer, sodium alginate. HPC and PVP (particularly, polyvinyl pyrrolidone K-90) are suitably used. The edible polymers include water-insoluble polymers such as water-insoluble ethyl cellulose, water-insoluble hydroxyethyl cellulose, shellac, cellulose acetate phthalate (CAP) and polyvinyl acetate, and water-swellable polymers such as Eudragit^{®}, hydroxypropylmethyl cellulose acetate succinate (HPMCAS) and hydroxypropylmethyl cellulose phthalate (HPMCP), as well as the above-mentioned edible water-soluble polymers. Although polyethylene glycol includes Macrogol 300 and Macrogol 400, Macrogol 400 is suitable. In the present invention, a sweetener and a flavor can be used, in addition to the above-mentioned ingredients. As the sweeteners, there can be used saccharine, saccharin sodium, aspartame, stevioside, sugar, fructose, glucose. As the flavors, various kinds of flavors can be used.

A nicotine-containing layer of the present invention comprises nicotine and an edible water-soluble polymer.

Nicotine used in the invention is desirably one having a grade of 99% or more. Low-grade nicotine is unsuitable because of its strong stimulation. The nicotine content in one preparation unit is from 0.1 to 5 mg. There is no particular limitation on the edible water-soluble polymer, a main adhesive ingredient, and examples thereof include hydroxypropylcellulose (HPC), hydroxypropylmethyl cellulose (HPMC), hydroxypropylethylcellulose (HPEC), methyl cellulose (MC), water-soluble ethyl cellulose (EC), water-soluble hydroxyethyl cellulose (HEC), carboxymethyl cellulose·sodium (CMC·Na), polyvinyl alcohol (PVA), polyvinyl pyrrolidone (PVP), a carboxyvinyl polymer, sodium alginate. HPC and PVP (particularly, polyvinyl pyrrolidone K-90) are suitably used. The compounding ratio of the edible water-soluble polymer is from 40 to 90% based on the whole preparation. That of the sweetener is preferably from 0.2 to 0.8% as well, the compounding ratio of the water-soluble and non-water-absorbing polysaccharide is preferably from 3 to 6% as well, that of the softener is preferably from 0.5 to 2% as well, and the compounding ratio of the edible polymer is preferably from 3 to 6% as well. Further, that of polyethylene glycol is preferably from 1 to 10% as well.

In this film preparation, the thickness of the coating layer is suitably from 5 to 30 µm, and the thickness of the nicotine-containing layer is suitably from 250 to 500 µm. The thickness as a whole is suitably from 260 to 560 µm. When the coating layer becomes too thick, the flexibility of the preparation is lost. Although it easily dissolves, some time is required because of its thickness, resulting in the difficulty of smoothly adhering to the inside of the oral cavity (upper jaw). Conversely, when it is too thin, unevenness is liable to occur in thickness in coating. In the extreme case, this introduces the possibility of some portions being not coated. In such a case, the protection of the nicotine-containing layer becomes insufficient. When the nicotine-containing layer becomes too thick, the time required for dissolution is unfavorably prolonged. Conversely, when it is thin, the duration is shortened, resulting in the possibility of the beneficial effect being not sufficiently exhibited.

As for the preparation like the present invention, there is no particular requirement on the time required for dissolution. However, the use of materials having properties as described above and the thickness as described above allow the preparation of the present invention to dissolve in 10 to 30 minutes. Faster than 10 minutes results in insufficient absorption of nicotine through the mucous membrane, whereas slower than 30 minutes results in the occurrence of various disadvantages.

The peeling strength in application to a Bakelite^{™} plate of the nicotine-containing layer and the coating layer of the film preparation of the present invention are from 3 to 10 minutes and from 0.5 to 3 minutes, respectively. The peeling strength in application to a Bakelite plate was measured in the following manner. A nicotine-containing layer patch or a coating layer patch is spread on a PET film having a thickness of 10 µm to prepare a film having a thickness of 100 µm. The film is cut to a size of 2 x 5 cm to prepare a test piece. A patch face of the test piece is uniformly impregnated with 200 µml of purified water, and then stuck on the Bakelite plate. A roller having a weight of 850 g is applied onto the Bakelite plate from above to adhere the patch face to the Bakelite plate. Then, a periphery of the test piece is pinched with a clip connected to a weight-measuring unit of a rheometer, and a test table is allowed to go down at a speed of 20 cm/min. Thus, the peeling strength of each patch to the Bakelite plate is determined (as the rheometer, Type RT-3020D-CW manufactured by Leotec Co. was used).

The peeling strength of the nicotine-containing layer in application to the Bakelite plate is suitably from 1 to 30 g/20 mm. There are the problems that less than 1 g/20 mm results in insufficient compatibility with the coating layer, whereas more than 30 g/20 mm results in the difficulty of production, the difficulty of separating from the base film when spread, and the difficulty of adhering to the inside of the oral cavity (upper jaw) even when tentatively used as a product.

Further, the peeling strength of the coating layer to the Bakelite plate is suitably from 100 to 500 g/20 mm. Less than 100 g/20 mm results in the difficulty of adhering to the inside of the oral cavity (upper jaw), whereas more than 500 g/20 mm results in the difficulty of production.

The solubility in water was measured in the following manner.

A nicotine-containing layer and a coating layer are each prepared so as to form a 100-µm film in the same manner as described above. These are cut to a size of 1.5 x 1.5 cm to prepare test pieces. Each test piece is taken, and tested at 50 revolutions with a sinker by The Pharmacopoea of Japan, the general test method, the elution test method, the second method, using 900 mL of purified water as a test solution. Complete dissolution is visually confirmed, and the time required for dissolution is taken as the solubility. The solubility in water of the nicotine-containing layer is suitably from 3 to 10 minutes. Faster than 3 minutes results in no expectation of the nicotine effect, whereas slower than 10 minutes results in the possibility of experiencing a feeling that something is wrong in the oral cavity. Further, the solubility in water of the coating layer is suitably from 0.5 to 3 minutes. Faster than 0.5 minute or slower than 3 minutes results in the difficulty of adhering to the inside of the oral cavity (upper jaw).

The pH of the film preparation of the present invention (a 0.1% aqueous solution) is suitably from 3 to 9. One sheet of this preparation is dissolved in 10 ml of purified water, and subjected to a measurement. When the pH is lower than 3 or higher than 9, stimulation to the mucous membrane is liable to occur.

The time required for dissolution of the film preparation of the present invention is suitably from 10 to 30 minutes as described above.

For the time required for dissolution, one sheet of this preparation was stuck on the upper jaw of each of healthy volunteers (7 volunteers), and the time required for complete dissolution was measured (sensory evaluation).

The strength of the film preparation of the present invention is suitably from 2 to 5 kg/20 mm.

This film preparation is cut to a size of 2 x 5 cm (measuring length: 3 cm) to prepare a test piece. A periphery (2 cm in width) of the test piece was connected to a weight measuring unit of a rheometer, and a test table is allowed to go down at a speed of 20 cm/min, thereby breaking this film preparation. The breaking strength required at that time is measured. When the strength is weaker than 2 kg/20 mm, the expected time required for dissolution is not obtained. Stronger than 5 kg/20 mm results in the possibility of the film cracking.

### Examples

Examples and reference examples are shown bellow.

### Example 1

To an appropriate amount of purified water, 79.5 g of pullulan, 20.0 g of D-sorbitol and 0.5 g of a sucrose fatty acid ester are added, and dissolved by stirring to prepare a coating layer solution. Separately, 2.0 g of nicotine, 4.0 g of titanium oxide, 8.0 g of Macrogol 400, 1.0 g of 1-menthol, 0.5 g of saccharin sodium, 0.1 g of a flavor and 84.4 g of hydroxypropylcellulose (L) are added to an appropriate amount of ethanol, and dissolved by stirring to prepare a nicotine-containing layer solution. Then, the coating layer solution is spread on a polyester release film, and dried to form a film having a thickness of about 10 µm. The nicotine-containing layer solution is spread thereon, and dried to prepare a film having a thickness of about 330 µm. Finally, the coating layer solution is spread thereon, and dried to form a film having a thickness of about 10 µm. The layers are thus laminated to prepare a film-shaped product having a thickness of about 350 µm as a whole. This was stamped out to a circular form having a diameter of 12 mm, thereby obtaining a nicotine-containing film preparation.

Hereinafter, Examples 2 and 3 and Comparative Examples 1 and 2 were carried out similarly to Example 1. Results thereof are shown in Table 1.

**Table 1**

| Layer | Name of Raw Material | | Example 1 | Example 2 | Example 3 | Comparative Example 1 | Comparative Example 2 |
|---|---|---|---|---|---|---|---|
| Coating Layer | Pullulan PI-20 | | 79.5 | | | | 22.0 |
| | D-sorbitol | | 20.0 | | | | 75.0 |
| | Sucrose Fatty Acid Ester | | 0.5 | | | | 3.0 |
| Nicotine-Containing Layer | Nicotine | | 2.0 | 2.0 | 2.0 | 2.0 | 2.0 |
| | Titanium Oxide | | 4.0 | | 4.0 | | |
| | PEG 400 | | 8.0 | 5.0 | 5.0 | 10.0 | 10.0 |
| | HPC-L | | 84.4 | 51.0 | 82.4 | 38.0 | 38.0 |
| | PVP K90 | | | 40.0 | | | |
| | PVP K30 | | | | | 50.0 | 50.0 |
| | Ethyl Cellulose | | | | | | |
| | 1-Menthol | | 1.0 | | 1.0 | | |
| | Saccharin Sodium | | 0.5 | | 0.5 | | |
| | Citric Acid | | | 2.0 | 5.0 | | |
| | Flavor | | 0.1 | | 0.1 | | |
| Claim 1 | Nicotine-Containing Layer | Peel strength (g/20 mm) | 5.6 | 5.0 | 5.3 | 35.0 | 35.0 |
| | | Solubility (min) | 5.0 | 3.8 | 5.0 | 2.8 | 2.8 |
| | Coating Layer | Peel strength (g/20 mm) | 296.7 | | | | 85.0 |
| | | Solubility (min) | 1.5 | | | | 0.4 |
| Claim 2 | PH | | 8.85 | 6.80 | 3.56 | 9.20 | 9.20 |
| Claim 3 | Nicotine Content (mg) | | 1.0 | | | | |
| Claim 4 | Time Required for Dissolution (min) | | 21.0 | 18.0 | 20.9 | 9.0 | 8.0 |
| Claim 5 | Strength (kg/20 mm) | | 2.8 | 2.6 | 3.0 | 1.8 | 1.7 |
| Claim 6 | Nicotine-Containing Layer (µm) | | 350 | 370 | 350 | 390 | 380 |
| | Coating Layer (µm) | | 10 | | | | |

### Comparative Example 1

The solubility of the nicotine-containing layer is high (2.8 minutes), so that the time required for dissolution becomes faster than the proper time (9.0 minutes) , which poses a problem with regard to sustained release. The pH is high, so that stimulation is given to the mucous membrane. The peeling strength is so high that the production thereof is difficult. For example, when spread on a PET film, it becomes difficult to be separated from the PET film.

### Comparative Example 2

The peel strength of the coating layer is low, and the adhesive force is low. Accordingly, adhesion to the upper jaw becomes difficult. The others are similar to Comparative Example 1.

### Effect of the Invention

The nicotine film preparation of the present invention is low in solubility, has a sustained release, and is moderate in the peeling strength of the nicotine-containing layer and the peeling strength of the coating layer, so that it easily adheres to the mucous membrane. Further, the nicotine film preparation of the present invention has a pH of 3 to 9, has no stimulation to the mucous membrane, is rich in flexibility and has elasticity, and has no feeling that something is wrong in application.

### Industrial Applicability

The nicotine film preparation of the present invention easily adheres to the mucous membrane, has a suitable sustained release of nicotine and is good in reproducibility thereof, is rich in flexibility and has elasticity, and has no feeling that something is wrong in application. Thus, the preparation is useful as a nicotine-containing film preparation for application to the oral mucous membrane, which is used as an aid for smoking cessation or moderation in smoking.

## Claims

1. A film preparation of a three-layer structure consisted of a nicotine-containing film layer and two coating layer films laminated respectively on both sides of the nicotine-containing layer film as a patch for the oral cavity, wherein
(1) the nicotine-containing film layer comprises nicotine and an edible water-soluble polymer and has a 180-degree peel strength in application to a Bakelite plate of 1 g to 30 g/20 mm and a time required for dissolution in water of 3 to 10 min as measured by dissolution of a 1.5 x 1.5 cm test piece of a 100 µm film in 900 ml of purified water at 50 revolutions with a sinker, and
(2) each of the coating layers comprises
(i)
(a) at least one of pullulan, a pullulan ether and a pullulan ester as a water-soluble and non-water-absorbing polysaccharide; or
(b) at least one of pullulan, a pullulan ether and a pullulan ester as a water-soluble and non-water-absorbing polysaccharide and an edible polymer; and
(ii) a softener, and
each of the coating layers has a 180-degree peel strength in application to a Bakelite plate of 100 g to 500g/20 mm and a time required for dissolution in water of 0.5 to 3 min as measured by dissolution of a 1.5 x 1.5 cm test piece of a 100 µm film in 900 ml of purified water at 50 revolutions with a sinker.

2. The film preparation according to claim 1, in which the pH of a 0.1% aqueous solution of the preparation is from 3 to 9.

3. The film preparation according to claim 1, in which the nicotine content in one preparation unit is from 0.1 to 5 mg.

4. The film preparation according to claim 1, in which the time required for dissolution in water of one preparation unit is from 10 minutes to 30 minutes as measured by dissolution of a 1.5 x 1.5 cm test piece of a 100 µm film in 900 ml of purified water at 50 revolutions with a sinker.

5. The film preparation according to claim 1, which has a film strength of 2 kg to 5 kg/20 mm.

6. The film preparation according to claim 1, in which the nicotine-containing layer has a thickness of 250 µm to 500 µm, the coating layer has a thickness of 5 µm to 30 µm, and a total of the three layers have a thickness of 260 µm to 560 µm.

7. The film preparation according to claim 1, in which the coating layer contains a sucrose fatty acid ester.

8. The film preparation according to claim 1, in which the nicotine-containing layer contains polyethylene glycol.

9. The film preparation according to claim 1, in which the water-soluble and non-water-absorbing polysaccharide is at least one of water-soluble starch, pullulan, a pullulan ether and a pullulan ester the softener is at least one of glycerol, ethylene glycol, propylene glycol, D-sorbitol, maltitol, mannitol and xylitol; the edible water-soluble polymer is at least one of hydroxypropylcellulose (HPC), hydroxypropylmethyl cellulose (HPMC), hydroxypropylethylcellulose (HPEC), methyl cellulose (MC), water-soluble ethyl cellulose (EC), water-soluble hydroxyethyl cellulose (HEC), carboxymethyl cellulose · sodium (CMC·Na), polyvinyl alcohol (PVA) , polyvinyl pyrrolidone (PVP), a carboxyvinyl polymer, sodium alginate and sodium acrylate; and the edible polymer is at least one of hydroxypropylcellulose (HPC), hydroxypropylmethyl cellulose (HPMC), hydroxypropyl- ethyl cellulose (HPEC), methyl cellulose (MC), ethyl cellulose (EC), hydroxyethyl cellulose (HEC), carboxymethyl cellulose· sodium (CMC·Na), polyvinyl alcohol (PVA), polyvinyl pyrrolidone (PVP), a carboxyvinyl polymer, sodium alginate, sodium acrylate, shellac, cellulose acetate phthalate (CAP), polyvinyl acetate, Eudragit^{®} , hydroxypropylmethyl cellulose acetate succinate (HPMCAS) and hydroxypropylmethyl cellulose phthalate (HPMCP).

10. The film preparation according to claim 1 or 9, wherein
the pullulan ether is selected from pullulan methyl ether, pullulan ethyl ether and pullulan propyl ether and the pullulan ester is selected from pullulan acetate and pullulan butyrate.

## Patentansprüche

1. Filmpräparat aus einer dreischichtigen Struktur, bestehend aus einer nicotinhaltigen Filmschicht und zwei Beschichtungsfilmschichten, die jeweils auf beide Seiten der nicotinhaltigen Filmschicht laminiert sind, als Pflaster für die Mundhöhle, wobei
(1) die nicotinhaltige Filmschicht Nicotin und ein verzehrfähiges wasserlösliches Polymer umfasst und in Anwendung auf eine Bakelitplatte eine 180-Grad-Schälfestigkeit von 1 g bis 30 g/20 mm aufweist und die zum Auflösen in Wasser erforderliche Zeit 3 bis 10 Minuten beträgt, gemessen durch Auflösen eines 1,5 x 1,5 cm großen Teststücks eines 100 µm dicken Films in 900 ml gereinigtem Wasser mit 50 Umdrehungen mit einem Ballaststück; und
(2) jede der Beschichtungsschichten Folgendes umfasst:
(i)
(a) Pullulan, einen Pullulanether und/oder einen Pullulanester als wasserlösliches und nichtwasserabsorbierendes Polysaccharid; oder
(b) Pullulan, einen Pullulanether und/oder einen Pullulanester als wasserlösliches und nichtwasserabsorbierendes Polysaccharid und ein verzehrfähiges Polymer; und
(ii) einen Weichmacher; und
jede der Beschichtungsschichten in Anwendung auf eine Bakelitplatte eine 180-Grad-Schälfestigkeit von 100 g bis 500 g/20 mm aufweist und die zum Auflösen in Wasser erforderliche Zeit 0,5 bis 3 Minuten beträgt, gemessen durch Auflösen eines 1,5 x 1,5 cm großen Teststücks eines 100 µm dicken Films in 900 ml gereinigtem Wasser mit 50 Umdrehungen mit einem Ballaststück.

2. Filmpräparat gemäß Anspruch 1, wobei der pH-Wert einer 0,1%igen wässrigen Lösung des Präparats 3 bis 9 beträgt.

3. Filmpräparat gemäß Anspruch 1, wobei der Nicotingehalt in einer Präparateinheit 0,1 bis 5 mg beträgt.

4. Filmpräparat gemäß Anspruch 1, wobei die zum Auflösen einer Präparateinheit in Wasser erforderliche Zeit 10 Minuten bis 30 Minuten beträgt, gemessen durch Auflösen eines 1,5 x 1,5 cm großen Teststücks eines 100 µm dicken Films in 900 ml gereinigtem Wasser mit 50 Umdrehungen mit einem Ballaststück.

5. Filmpräparat gemäß Anspruch 1, das eine Filmfestigkeit von 2 kg bis 5 kg/20 mm aufweist.

6. Filmpräparat gemäß Anspruch 1, wobei die nicotinhaltige Schicht eine Dicke von 250 µm bis 500 µm hat, die Beschichtungsschicht eine Dicke von 5 µm bis 30 µm hat und die drei Schichten insgesamt eine Dicke von 260 µm bis 560 µm haben.

7. Filmpräparat gemäß Anspruch 1, wobei die Beschichtungsschicht einen Saccharosefettsäureester enthält.

8. Filmpräparat gemäß Anspruch 1, wobei die nicotinhaltige Schicht Polyethylenglycol enthält.

9. Filmpräparat gemäß Anspruch 1, wobei es sich bei dem wasserlöslichen und nichtwasserabsorbierenden Polysaccharid um wenigstens eines von wasserlösliche Stärke, Pullulan, Pullulanether und Pullulanester handelt, es sich bei dem Weichmacher um wenigstens eines von Glycerin, Ethylenglycol, Propylenglycol, D-Sorbit, Maltit, Mannit und Xylit handelt, es sich bei dem verzehrfähigen wasserlöslichen Polymer um wenigstens eines von Hydroxypropylcellulose (HPC), Hydroxypropylmethylcellulose (HPMC), Hydroxypropylethylcellulose (HPEC), Methylcellulose (MC), wasserlösliche Ethylcellulose (EC), wasserlösliche Hydroxyethylcellulose (HEC), Carboxymethylcellulose-Natrium (CMC·Na), Polyvinylalkohol (PVA), Polyvinylpyrrolidon (PVP), ein Carboxyvinylpolymer, Natriumalginat und Natriumacrylat handelt und es sich bei dem verzehrfähigen Polymer um wenigstens eines von Hydroxypropylcellulose (HPC), Hydroxypropylmethylcellulose (HPMC), Hydroxypropylethylcellulose (HPEC), Methylcellulose (MC), Ethylcellulose (EC), Hydroxyethylcellulose (HEC), Carboxymethylcellulose-Natrium (CMC·Na), Polyvinylalkohol (PVA), Polyvinylpyrrolidon (PVP), ein Carboxyvinylpolymer, Natriumalginat, Natriumacrylat, Schellack, Celluloseacetatphthalat (CAP), Polyvinylacetat, Eudragit, Hydroxypropylmethylcelluloseacetatsuccinat (HPMCAS) und Hydroxypropylmethylcellulosephthalat (HPMCP) handelt.

10. Filmpräparat gemäß Anspruch 1 oder 9, wobei der Pullulanether aus Pullulanmethylether, Pullulanethylether und Pullulanpropylether ausgewählt ist und der Pullulanester aus Pullulanacetat und Pullulanbutyrat ausgewählt ist.

## Revendications

1. Préparation pour film présentant une structure à trois couches composée d'une couche de film contenant de la nicotine et de deux couches de film de revêtement stratifiées respectivement des deux côtés de la couche de film contenant de la nicotine pour constituer un timbre pour la cavité buccale, dans laquelle
(1) la couche de film contenant de la nicotine comprend de la nicotine et un polymère soluble dans l'eau comestible et a une résistance au pelage de 180 degrés lors de son application sur une plaque de bakélite de 1 g à 30 g / 20 mm et une solubilité dans l'eau de 3 à 10 min, mesuré par la dissolution d'un échantillon de 1,5 x 1,5 cm d'un film de 100 µm dans 900 ml d'eau purifiée à 50 tours à l'aide d'un plongeur, et
(2) chacune des couches de revêtement comprend
(i)
(a) au moins un élément parmi le pullulane, un éther de pullulane et un ester de pullulane en tant que polysaccharide soluble dans l'eau et n'absorbant pas l'eau ; ou
(b) au moins un élément parmi le pullulane, un éther de pullulane et un ester de pullulane en tant que polysaccharide soluble dans l'eau et n'absorbant pas l'eau et un polymère comestible ; et
(ii) un adoucissant, et
chacune des couches de revêtement a une résistance au pelage de 180 degrés lors de son application sur une plaque de bakélite de 100 g à 500 g / 20 mm et nécessite de 0,5 à 3 min pour se dissoudre dans l'eau, mesuré par la dissolution d'un échantillon de 1,5x 1,5 cm d'un film de 100 µm dans 900 ml d'eau purifiée à 50 tours à l'aide d'un plongeur.

2. Préparation pour film selon la revendication 1, dans laquelle le pH d'une solution aqueuse à 0,1 % de la préparation est compris dans la plage de 3 à 9.

3. Préparation pour film selon la revendication 1, dans laquelle la teneur en nicotine dans une unité de préparation est comprise dans la plage de 0,1 à 5 mg.

4. Préparation pour film selon la revendication 1, dans laquelle le temps nécessaire pour la dissolution dans l'eau d'une unité de préparation est compris dans la plage de 10 minutes à 30 minutes, mesuré par la dissolution d'un échantillon de 1,5 x 1,5 cm d'un film de 100 µm dans 900 ml d'eau purifiée à 50 tours à l'aide d'un plongeur.

5. Préparation pour film selon la revendication 1 ayant une résistance de film de 2 kg à 5 kg / 20 mm.

6. Préparation pour film selon la revendication 1, dans laquelle la couche contenant de la nicotine a une épaisseur de 250 µm à 500 µm, la couche de revêtement a une épaisseur de 5 µm à 30 µm et l'ensemble des trois couches a une épaisseur de 260 µm à 560 µm.

7. Préparation pour film selon la revendication 1, dans laquelle la couche de revêtement contient un ester d'acide gras de saccharose.

8. Préparation pour film selon la revendication 1, dans laquelle la couche contenant de la nicotine contient du polyéthylène glycol.

9. Préparation pour film selon la revendication 1, dans laquelle le polysaccharide soluble dans l'eau et n'absorbant pas l'eau est au moins un élément parmi l'amidon soluble dans l'eau, le pullulane, un éther de pullulane et un ester de pullulane ;
l'adoucissant est au moins un élément parmi le glycérol, l'éthylène glycol, le propylène glycol, le D-sorbitol, le maltitol, le mannitol et le xylitol ; le polymère soluble dans l'eau comestible est au moins un élément parmi l'hydroxypropylcellulose (HPC), l'hydroxypropylméthylcellulose (HPMC), l'hydroxypropyléthylcellulose (HPEC), la méthylcellulose (MC), l'éthylcellulose soluble dans l'eau (EC), l'hydroxyéthylcellulose soluble dans l'eau (HEC), le carboxyméthylcellulose sodium (CMC.Na), l'alcool polyvinylique (PVA), le pyrrolidone polyvinylique (PVP), un polymère de carboxyvinyle, un alginate de sodium et un acrylate de sodium ; et le polymère comestible est au moins un élément parmi l'hydroxypropylcellulose (HPC), l'hydroxypropylméthylcellulose (HPMC), l'hydroxypropyéthylcellulose (HPEC), la méthylcellulose (ME), l'éthylcellulose (EC), l'hydroxyéthylcellulose (HEC), le carboxyméthylcellulose sodium (CMC.Na), l'alcool polyvinylique (PVA), le pyrrolidone polyvinylique (PVP), un polymère de carboxyvinyle, un alginate de sodium, un acrylate de sodium, du shellac ; un cellulose acétate phthalate (CAP), un acétate polyvinylique, de l'Eudragit^{®}, un hydroxypropylméthylcellulose acétate succinate (HPMCAS) et un hydroxypropylméthylcellulose phthalate (HPMCP).

10. Préparation pour film selon les revendications 1 à 9, dans laquelle
l'éther de pullulane est sélectionné parmi l'éther de pullulane méthylé, l'éther de pullulane éthylé et l'éther de pullulane propylé et l'ester de pullulane est sélectionné parmi l'acétate de pullulane et le butyrate de pullulane.
